# EUROPÄISCHE PATENTSCHRIFT

(11) **EP 0 533 967 B1**
(45) Veröffentlichungstag und Bekanntmachung des Hinweises auf die Patenterteilung: **27.11.1996**
(21) Anmeldenummer: 91116160.2
(22) Anmeldetag: 23.09.1991
(51) Int. Cl.: A61N 1/08, A61N 1/39, A61N 1/37

(54) **Schutzanordnung für einen implantierbaren Defibrillator**
Protection device for an implantable defibrillator
Dispositif de sécurité pour défibrillateur implantable

(43) Veröffentlichungstag der Anmeldung: 31.03.1993
(73) Patentinhaber: Pacesetter AB, 171 95 Solna (SE)
(72) Erfinder: Hirschberg, Jakub, Dipl.-Ing., S-183 44 Täby (SE); Strandberg, Hans, Dipl.-Ing., S-172 36 Sundbyberg (SE)
(74) Vertreter: Lettström, Richard Wilhelm

(56) Entgegenhaltungen:
- EP-A- 0 315 368
- FR-A- 2 053 539

## Beschreibung

Die Erfindung betrifft eine Hochspannungsschutzanordnung für einen implantierbaren Defibrillator, der ein Gehäuse mit Anschlußbuchsen zum Anschluß von Elektroden aufweist.

Implantierbare Defibrillatoren enthalten bekannterweise einen in dem Gehäuse des Defibrillators angeordneten Hochspannungsgenerator, der auf Anforderung einen Hochspannungsimpuls an den Anschlußbuchsen erzeugt, der bei dem implantierten Defibrillator über Elektroden an das zu defibrillierende Herz abgegeben wird. Die Anforderung zur Abgabe eines Hochspannungsimpulses kann automatisch nach Auswertung von meßtechnisch erfaßten physiologischen Parametern erfolgen, die einen Fibrillationszustand des Herzens signalisieren, oder beispielsweise für Testzwecke über ein Programmiergerät von außen her erfolgen.

Um bei der Herstellung und beim späteren Hantieren des Defibrillators im Zusammenhang mit seiner Implantation zu verhindern, daß die den Defibrillator hantierende Person aufgrund irgendeines Fehlers einen elektrischen Schlag erhält, ist gemäß der Erfindung für den implantierbaren Defibrillator der eingangs angegebenen Art eine Schutzanordnung vorgesehen, die aus einem mit dem Defibrillatorgehäuse lösbar verbindbaren Steckerteil mit Anschlußstiften besteht, die unter Herstellung einer elektrisch leitenden Verbindung in die Anschlußbuchsen eingreifen und innerhalb des Steckerteils direkt über einen elektrischen Widerstand miteinander verbunden sind.

Es ist zwar aus der US-A-4 830 005 ein ähnliches Steckerteil für einen Herzschrittmacher bekannt, jedoch ist bei dem bekannten Steckerteil zwischen den Anschlußstiften ein Widerstand in Reihe mit einem Magnetschalter angeordnet, der zu Testzwecken mittels eines äußeren Prüfmagenten geschlossen werden kann. Dabei geht es also nicht um einen Schutz gegen Hochspannung, sondern darum, mit dem Widerstand die Herzimpedanz zu simulieren, um den Herzschrittmacher vor einer Implantation auf seine Funktionsfähigkeit überprüfen zu können. Ein Hochspannungsschutz wird mit dieser bekannten Anordnung nicht erzielt, weil der Magnetschalter im Normalfall geöffnet ist.

Aus der US-A-3 798 542 ist eine Anordnung bestehend aus einer Widerstandskaskade mit einer Glimmlampe als Anzeigeelement zum Testen der Ausgangsenergie von Defibrillatoren bekannt. Die bekannte Testschaltung weist Kontaktflächen auf, an die die Elektroden des Defibrillators flächig angelegt werden, um anschließend die Abgabe eines Hochspannungsimpulses durch den Defibrillator auszulösen.

Im Unterschied zu den bekannten Prüfschaltungen wird mit Hilfe der erfindungsgemäßen Schutzanordnung ein effektiver Hochspannungsschutz gewährleistet, wobei nicht nur die Anschlußbuchsen des Defibrillators gegen zufälliges Berühren geschützt sind, sondern auch im Falle eines Fehlers die zwischen den Anschlußbuchsen erzeugte Hochspannungsenergie auf unschädlichem Wege in dem Widerstand absorbiert wird. Ein weiterer Vorteil besteht darin, daß ein mögliches Kurzschließen der Anschlußbuchsen und damit eine Beschädigung des Defibrillators durch einen Hochspannungskurzschluß verhindert wird. Bei Defibrillatoren, die mehr als zwei hochspannungsführende Anschlußbuchsen aufweisen, besteht der Widerstand aus mehreren Einzelelementen, die zwischen den ebenfalls mehr als zwei Anschlußstiften angeordnet sind. Zum Anschließen der Elektroden oder bei Ausführung bestimmter Tests, in denen die hochspannungsführenden Anschlußbuchsen zugänglich sein müssen, wird das Steckerteil einfach von dem Gehäuse des Defibrillators entfernt. Im übrigen kann aber der Defibrillator bei angestecktem Steckerteil getestet werden, wobei der Widerstand als Last dient. Dies ist insbesondere dann von Vorteil, wenn der Defibrillator bereits steril verpackt ist, weil der Test durchgeführt werden kann, ohne daß die Verpackung geöffnet werden muß.

Eine sichere Absorbtion der im Fehlerfalle von dem Defibrillator an den Anschlußbuchsen abgegebenen Hochspannungsenergie wird in vorteilhafter Weise dadurch erreicht, daß der Widerstandswert des Widerstandes in der Größenordnung von 10 Ω liegt.

Das Steckerteil besteht vorzugsweise aus einer isolierenden Kunststoffmasse, in die der Widerstand eingegossen ist. Auf diese Weise wird ein sicherer Berührschutz bei gleichzeitiger einfacher Herstellbarkeit des Steckerteils gewährleistet.

Um das Steckerteil beim Implantieren des Defibrillartors leicht von diesen trennen zu können, ist dieses vorzugsweise mit seitlichen Griffmulden versehen.

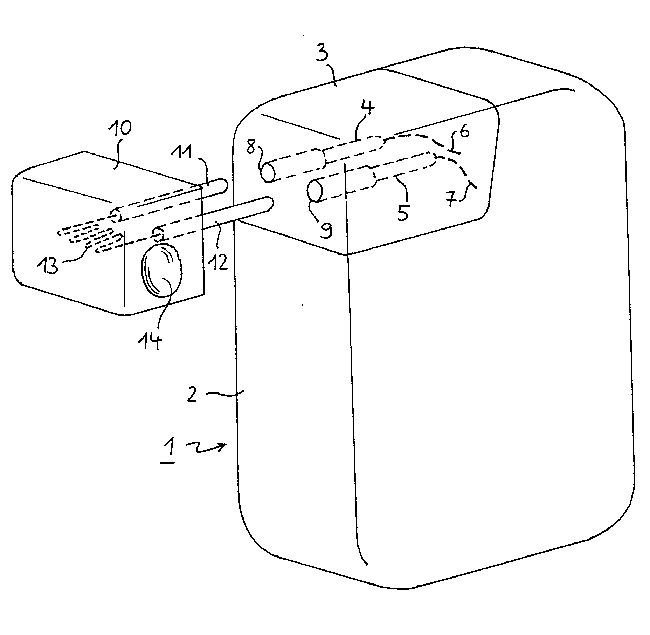

Zu Erläuterung der Erfindung wird im Folgenden auf die Figur der Zeichnung Bezug genommen, die einen implantierbaren Defibrillator zusammen mit einem Ausführungsbeispiel der für ihn vorgesehenen erfindungsgemäßen Schutzanordnung zeigt.

Der dargestellte Defibrillator 1 weist ein metallenes Gehäuse 2 auf, das für den Anschluß von hier nicht gezeigten Elektroden mit einem Anschlußteil 3 aus Kunststoff versehen ist. In dem Kunststoffteil 3 sind zwei Anschlußbuchsen 4 und 5 eingelassen, die über Verbindungsleitungen 6 und 7 mit einem hier nicht gezeigten Hochspannungsgenerator im Inneren des Gehäuses 2 verbunden sind. Die Anschlußbuchsen 4 und 5 sind durch Bohrungen 8 und 9 in dem Kunststoffteil 3 von außen her zugänglich.

Mit 10 ist ein Steckerteil bezeichnet, das entsprechend der Anzahl der Anschlußbuchsen 4 und 5 zwei Anschlußstifte 11 und 12 in gleicher Anordnung aufweist. Die Anschlußstifte 11 und 12 sind im Inneren des Steckerteils 10 über einen Drahtwiderstand mit einem Widerstandswert in der Größenordnung von 10 Ω miteinander verbunden. Das Steckerteil 10 besteht aus einer isolierenden Kunststoffmasse, in die der Drahtwiderstand 13 und die mit diesem verbundenen Endteile der Anschlußstifte 11 und 12 eingegossen sind. Zur Erleichterung der Handhabbarkeit des Steckerteils 10 beim Aufstecken an das Gehäuse 2 bzw. beim Lösen vom Gehäuse 2 ist das Steckerteil 10 mit seitlichen Griffmulden,z.B. 14, versehen.

Solange der Defibrillator 1 nicht implantiert wird oder zu Testzwecken über die Anschlußbuchsen 4 und 5 mit Elektrodenleitungen verbunden wird, ist das Steckerteil 10 mit den Anschlußstiften 11 und 12 in die Anschlußbuchsen 4 und 5 eingesetzt. Sollte dann auf Grund irgendeinens Fehlers in der Steuerung und Hochspannungserzeugung des Defibrillators 1 an den Anschlußbuchsen 4 und 5 eine Hochspannung auftreten, so wird die zugehörige Hochspannungsenergie auf eine für die den Defibrillator 1 hantierende Person unschädlichliche Weise in dem Widerstand 13 absorbiert. Das Steckerteil 10 wird im übrigen erst zum Anschließen der Elektroden an den implantierten Defibrillator 1 von diesen entfernt.

## Patentansprüche

1. Hochspannungsschutzanordnung für einen implantierbaren Defibrillator (1), der ein Gehäuse (2) mit Anschlußbuchsen (4, 5) zum Anschluß von Elektroden aufweist, **dadurch gekennzeichnet,** daß diese Anordnung besteht aus einem mit dem Gehäuse (2) lösbar verbindbaren Steckerteil (10) mit unter Herstellung einer elektrisch leitenden Verbindung in die Anschlußbuchsen (4,5) eingreifenden Anschlußstiften (11,12), die innerhalb des Steckerteils (10) direkt mit einem elektrischen Widerstand (13) verbunden sind.

2. Hochspannungsschutzanordnung nach Anspruch 1, **dadurch gekennzeichnet**, daß der Widerstandswert des Widerstandes (13) in der Größenordnung von 10 Ω liegt.

3. Hochspannungsschutzanordnung nach Ansprunch 1 oder 2, **dadurch gekennzeichnet**, daß das Steckerteil (10) aus einer isolierenden Kunststoffmasse besteht, in die der Widerstand (13) eingegossen ist.

4. Hochspannungsschutzanordnung nach einem der Ansprüche 1 bis 3, **dadurch gekennzeichnet**, daß das Steckerteil (10) mit seitlichen Griffmulden (14,15) versehen ist.

## Claims

1. High-voltage protection device for an implantable defibrillator (1) which has a housing (2) with terminal bushings (4, 5) for the connection of electrodes, characterized in that this device comprises a plug part (10) which can be releasably connected to the housing (2), the plug part having terminal pins (11, 12) engaging into the terminal bushings (4, 5) with production of an electroconductive connection, the pins being connected within the plug part (10) directly to an electrical resistor (13).

2. High-voltage protection device according to claim 1, characterized in that the resistance value of the resistor (13) lies in the order of magnitude of 10 Ω.

3. High-voltage protection device according to claim 1 or 2, characterized in that the plug part (10) comprises an insulating plastics mass into which the resistor (13) is poured.

4. High-voltage protection device according to one of claims 1 to 3, characterized in that the plug part (10) is provided with lateral recessed grips (14, 15).

## Revendications

1. Dispositif de protection haute tension pour un défibrillateur (1) pouvant être implanté, qui comporte un boîtier (2) muni de douilles (4,5) de raccordement pour le raccordement d'électrodes caractérisé en ce que ce dispositif est constitué d'une partie (10) d'enfichage pouvant être reliée de manière amovible au boîtier (2), munie de broches (11,12) de raccordement pénétrant dans les douilles (4,5) de raccordement avec formation d'une liaison conductrice de l'électricité, ces broches étant reliées directement à une résisance (13) électrique à l'intérieur de la partir (10) d'enfichage.

2. Dispositif de protection haute tension suivant la revendication 1, caractérisé en ce que la valeur de la résistance (13) est de l'ordre de grandeur de 10Ω.

3. Dispositif de protection haute tension suivant la revendication 1 ou 2, caractérisé en ce que la partie (10) d'enfichage est constituée d'une matière plastique isolante, dans laquelle la résistance (13) est enrobée.

4. Dispositif de protection haute tension suivant l'une des revendications 1 à 3 caractérisé en ce que la partie (10) d'enfichage est munié de cuvettes (14,15) de préhension latérales.
